**Europäisches Patentamt**

**European Patent Office**

**Office européen des brevets**

(19)

(11) Veröffentlichungsnummer: **0 036 578**
A1

(12) ## EUROPÄISCHE PATENTANMELDUNG

(21) Anmeldenummer: **81101842.3**

(22) Anmeldetag: **12.03.81**

(51) Int. Cl.³: **A 61 B  6/02,** A 61 B  6/00

(30) Priorität: **17.03.80  DE 3010230**

(43) Veröffentlichungstag der Anmeldung: **30.09.81**
**Patentblatt 81/39**

(84) Benannte Vertragsstaaten: **DE FR GB**

(71) Anmelder: **SIEMENS AKTIENGESELLSCHAFT Berlin
und München, Postfach 22 02 61,
D-8000 München 22 (DE)**

(72) Erfinder: **Pfeiler, Manfred, Dr.,
Ludwig-Thoma-Strasse 25, D-8520 Erlangen (DE)**
Erfinder: **Tschunt, Edgar, Im Winkel 9, D-8521 Rathsberg
(DE)**

(54) **Strahlendiagnostikeinrichtung.**

(57)  Die Erfindung bezieht sich auf eine Strahlendiagnostikeinrichtung mit einer Patientenliege (9), mit einer Strahlenmeßanordnung (4, 5) aus einer Strahlenquelle (4), die
ein das Aufnahmeobjekt durchdringendes, fächerförmiges
Röntgenstrahlenbündel (11) erzeugt, und einem Strahlenempfänger (5), der aus einer Reihe von Detektoren besteht, die an einer Signalverarbeitungsschaltung angeschlossen sind, sowie mit Mitteln zur Erzeugung einer Relativbewegung zwischen der Patientenliege (9) und der
Strahlenmeßanordnung (4, 5) in Liegenlängsrichtung für
die Erzeugung eines Schattenbildes aus den Detektor-
Ausgangssignalen durch die Signalverarbeitungsschaltung.
Die Strahlenquelle (4) und der Strahlenempfänger (5)
sind im Bereich der Enden an einem C-förmigen Träger
(2) befestigt, der um die Längsachse der Patientenliege
(9) oder eine dazu parallele Achse (10) drehbar gelagert
ist.

SIEMENS AKTIENGESELLSCHAFT                  Unser Zeichen
Berlin und München                          VPA 80 P 5034   E


Strahlendiagnostikeinrichtung

Die Erfindung betrifft eine Strahlendiagnostikeinrichtung mit einer Patientenliege, mit einer Strahlenmeßanordnung aus einer Strahlenquelle, die ein das Aufnahmeobjekt durchdringendes, fächerförmiges Strahlenbündel erzeugt und einem Strahlenempfänger, der aus einer Reihe von Detektoren besteht, die an einer Signalverarbeitungsschaltung angeschlossen sind, sowie mit Mitteln zur Erzeugung einer Relativbewegung zwischen der Patientenliege und der Strahlenmeßanordnung in Liegenlängsrichtung für die Erzeugung eines Schattenbildes aus den Detektor-Ausgangssignalen durch die Signalverarbeitungsschaltung.

Eine Strahlendiagnostikeinrichtung dieser Art ist in der DE-OS 26 13 809 beschrieben. Bei der bekannten Strahlendiagnostikeinrichtung handelt es sich um einen Computertomographen, der mit Mitteln ergänzt ist, die es erlauben, ein Schattenbild eines bestimmten Bereiches eines Patienten anzufertigen. Für die Anfertigung eines Computertomogrammes wird die Meßanordnung um die Liegenlängsachse oder eine dazu parallele Achse gedreht. Aus den Ausgangssignalen der Detektoren berechnet ein Computer dabei die Schwächungswerte von in einer Matrix angeordneten Bildpunkten, die dann als Bild der untersuchten Transversalschicht des Patienten wiedergegeben werden können. Für die Erzeugung eines Schattenbildes wird die Meßanordnung gegen Drehung verriegelt und es erfolgt eine Relativbewegung zwischen der Patientenliege und der Meßanordnung in Liegenlängs-richtung.

Tp 5 Kli / 9.3.1981

Der Erfindung liegt die Aufgabe zugrunde, eine Strahlendiagnostikeinrichtung der eingangs genannten Art zu schaffen, bei der die Strahlenquelle und der Strahlenempfänger so gelagert sind, daß es in einfacher Weise möglich ist, den Patienten von der Seite auf die Patientenliege zu legen und dann ein Schattenbild bei einer wählbaren Projektion anzufertigen.

Diese Aufgabe ist erfindungsgemäß dadurch gelöst, daß die Strahlenquelle und der Strahlenempfänger im Bereich der Enden an einem C-förmigen Träger befestigt sind, der um die Längsachse der Patientenliege oder eine dazu parallele Achse drehbar gelagert ist. Bei der erfindungsgemäßen Strahlendiagnostikeinrichtung ist ein C-förmiger, also offener Träger für die Strahlenmeßanordnung vorhanden, der in eine Lage verdreht werden kann, in der ein seitlicher Zugang zur Patientenliege und damit ein seitliches Auflegen des Patienten auf die Patientenliege möglich ist. Durch Verdrehung kann dabei die jeweilige Projektion für die Anfertigung eines Schattenbildes gewählt werden.

Die Erfindung ist nachfolgend anhand eines in der Zeichnung dargestellten Ausführungsbeispiels näher erläutert. Es zeigen:

Fig. 1 eine Seitenansicht einer Strahlendiagnostikeinrichtung nach der Erfindung und

Fig. 2 eine Ansicht der Strahlendiagnostikeinrichtung gemäß Fig. 1 von vorne.

In den Figuren ist an einem Geräterahmen 1 ein C-förmiger Träger 2 in Rollen 3 drehbar gelagert, der an seinen Enden eine Röntgenröhre 4 und einen Strahlenempfänger 5 trägt. Der Träger 2 ist durch ein Gegengewicht 6 ausgewuchtet. Der Rahmen 1 besitzt eine Aussparung 7, in der eine auf einem Sockel 8 angeordnete Patientenliege 9 längsverschiebbar angeordnet ist. Der Träger 2 ist um eine parallel zur Längsachse der Patientenliege 9 verlaufende Achse 10 drehbar.

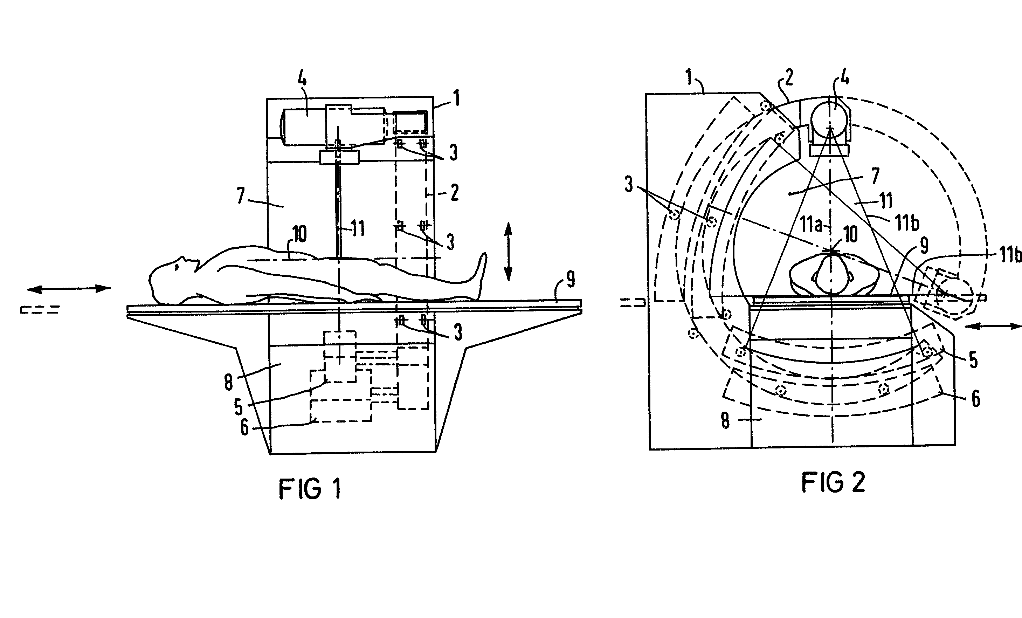

Die Röntgenröhre 4 erzeugt ein den Patienten durchdringendes fächerförmiges Röntgenstrahlenbündel 11, das eine Transversalschicht des Patienten vollkommen durchsetzt, wie dies aus der Fig. 2 hervorgeht. Der Strahlenempfänger 5 besteht aus einer Reihe von Detektoren, die an einer nicht dargestellten Signalverarbeitungsschaltung angeschlossen sind.

Zur Erzeugung eines Schattenbildes wird zunächst die gewünschte Projektion gewählt. Bei dem Beispiel stehen zwei mögliche Projektionen zur Verfügung, nämlich eine Projektion, bei der der Träger 2, die Röntgenröhre 4 und der Strahlenempfänger 5 ihre voll ausgezogen gezeichnete Stellung einnehmen, und eine zweite Projektion, bei der diese Teile ihre gestrichelt gezeichnete Stellung einnehmen (Fig. 2). In der ersten Stellung steht der Zentralstrahl 11a des Röntgenstrahlenbündels 11 senkrecht auf der Oberfläche der Patientenliege 9, während in der zweiten Stellung der rechte Randstrahl 11b des Röntgenstrahlenbündels 11 diese Oberfläche berührt.

Nach der Wahl der gewünschten Projektion wird der Träger 2 gegen Drehung arretiert und die Patientenliege 9 in ihrer Längsrichtung um ein vorbestimmtes Maß, das dem gewünschten Bildbereich entspricht, verschoben. Dabei wird die Röntgenröhre 4 z.B. gepulst. Bei jedem Röntgenstrahlenimpuls oder bei kontinuierlicher Strahlung nach einem Zeitintervall, das Bruchteil der Verschiebungszeit ist, werden die Ausgangssignale der Detektoren des Strahlenempfängers 5 von der Signalverarbeitungsschaltung abgefragt und gespeichert. Nach Beendigung eines Verschiebungsvorganges, d.h. nach Aufnahme des gewünschten Bildes, liegen die Informationen vorbestimmter Bildpunkte zeilenweise gespeichert vor. Jede Bildzeile wird dabei während eines Röntgenstrahlenimpulses aufgenommen. Die Bildpunkte pro Bildzeile entsprechen der Anzahl der Detektoren des Strahlenempfängers 5. Man erhält daher für vorbestimmte Stellungen der Patientenliege 9 Meßwerte, die die Schwächung der Röntgenstrahlung beim Durchtritt durch den Patienten charakterisieren. Das entsprechende Röntgenschattenbild kann dann auf einem Sichtgerät wiedergegeben werden. Jeder Bildzeile entspricht dabei eine Abfrage des Strahlenempfängers 5 bei einem Röntgenstrahlenimpuls. Die Pulsfrequenz und die Geschwindigkeit, mit der die Patientenliege 9 mit dem Patienten bewegt wird, sind so aufeinander abgestimmt, daß die örtliche Auflösung in Längsrichtung des Patienten eine gewünschte Größe hat.

Aufgrund der Tatsache, daß der Träger 2 in seiner in der Fig. 2 voll ausgezogenen Stellung einen seitlichen Zugang zur Patientenliege 9 ermöglicht, ist es in einfacher Weise möglich, den Patienten von der Seite her auf die Liege 9 zu legen. Er braucht also nicht in seiner Längsrichtung in die Aussparung 7 hineingeschoben zu werden.

Die beiden möglichen Projektionen, die in der Fig. 2 dargestellt sind, erlauben die Durchstrahlung des Patienten, ohne daß strahlenundurchlässige Teile der Patientenliege 9 oder des Sockels 8 von Strahlung durchsetzt werden, denn die Patientenliege 9 kann aus strah-

0036578

lendurchlässigem Material bestehen und die gegebenenfalls strahlenundurchlässigen Führungsteile können am
Rand der Patientenliege 9 angeordnet werden, so daß sie
in keiner der gezeichneten Projektionen von Röntgenstrahlung durchsetzt werden.

2 Figuren
1 Patentanspruch

Patentanspruch

Strahlendiagnostikeinrichtung mit einer Patientenliege, mit einer Strahlenmeßanordnung aus einer Strahlenquelle, die ein das Aufnahmeobjekt durchdringendes, fächerförmiges Strahlenbündel erzeugt und einem Strahlenempfänger, der aus einer Reihe von Detektoren besteht, die an einer Signalverarbeitungsschaltung angeschlossen sind, sowie mit Mitteln zur Erzeugung einer Relativbewegung zwischen der Patientenliege und der Strahlenmeßanordnung in Liegenlängsrichtung für die Erzeugung eines Schattenbildes aus den Detektor-Ausgangssignalen durch die Signalverarbeitungsschaltung, d a d u r c h   g e k e n n z e i c h n e t ,   daß die Strahlenquelle (4) und der Strahlenempfänger (5) im Bereich der Enden an einem C-förmigen Träger (2) befestigt sind, der um die Längsachse der Patientenliege (9) oder eine dazu parallele Achse drehbar gelagert ist.

0036578

1/1

FIG 2

FIG 1

| | Europäisches Patentamt | **EUROPÄISCHER RECHERCHENBERICHT** | 0036578 |
|---|---|---|---|

Nummer der Anmeldung

EP 81 10 1842

| **EINSCHLÄGIGE DOKUMENTE** | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| DA | <u>DE - A - 2 613 809</u> (SIEMENS A.G.)<br><br>* Seite 4, Zeile 8 - Seite 5, Zeile 6 und Abbildung 1 *<br><br>-- | 1 | A 61 B 6/02<br>6/00 |
| X | <u>GB - A - 2 028 053</u> (SIEMENS A.G.)<br><br>* Zusammenfassung; Seite 2, Zeile 66 - Seite 3, Zeile 19 und Abbildungen 3-8 *<br><br>-- | 1 | |
| X | <u>DE - A - 2 653 465</u> (PHILIPS MEDICAL SYSTEMS INC.)<br><br>* Seite 35, Zeile 4 - Seite 36, Zeile 12; Seite 52, Zeile 16- Seite 53, Zeilen 17 und Abbildungen 3,6 *<br><br>-- | 1 | RECHERCHIERTE SACHGEBIETE (Int. Cl.³)<br><br>A 61 B 6/02<br>6/00 |
| X | <u>EP - A - 0 002 088</u> (PHILIPS MEDICAL SYSTEMS INC.)<br><br>* Zusammenfassung; Seite 12, Zeilen 10-35; Seite 15, Zeile 18 - Seite 16, Zeile 1 und Abbildungen 1,3 *<br><br>-- | 1 | |
| X | <u>FR - A - 2 269 074</u> (REALISATIONS ULTRASONIQUES)<br><br>* Seite 1, Zeilen 17-24; Seite 2, Zeilen 18-36; Seite 4, Zeilen 15-40 und Abbildungen 1,2 *<br><br>-- | 1 | KATEGORIE DER GENANNTEN DOKUMENTE<br><br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | <u>US - A - 4 118 631</u> (R.J. FROGGATT/ EMI LTD.)<br><br>./. | 1 | |

| X | Der vorliegende Recherchenbericht wurde für alle Patentansprüche erstellt. | &: Mitglied der gleichen Patent-familie, übereinstimmendes Dokument |
|---|---|---|

| Recherchenort | Abschlußdatum der Recherche | Prüfer |
|---|---|---|
| Den Haag | 26-06-1981 | RIEB |

EPA form 1503.1 06.78

**Europäisches Patentamt**

| | EINSCHLÄGIGE DOKUMENTE | | KLASSIFIKATION DER ANMELDUNG (Int.Cl. ) |
|---|---|---|---|
| Kategorie | Kennzeichnung des Dokuments mit Angabe, soweit erforderlich, der maßgeblichen Teile | betrifft Anspruch | |
| | * Zusammenfassung; Spalte 3, Zeilen 5-25; und Abbildung 4 * | | |
| | ---- | | |
| | | | **RECHERCHIERTE SACHGEBIETE (Int. Cl. )** |

EPA Form 1503.2  06.78